(19) Europäisches Patentamt
European Patent Office
Office européen des brevets

(11) Publication number: 0 055 626
B1

(12) **EUROPEAN PATENT SPECIFICATION**

(45) Date of publication of patent specification: **02.05.85**

(51) Int. Cl.$^4$: **C 07 D 307/52** // A61K31/34

(21) Application number: **81306165.2**

(22) Date of filing: **30.12.81**

(54) **Process for the preparation of a furan derivative.**

(30) Priority: **30.12.80 GB 8041492**

(43) Date of publication of application:
**07.07.82 Bulletin 82/27**

(45) Publication of the grant of the patent:
**02.05.85 Bulletin 85/18**

(84) Designated Contracting States:
**AT BE CH DE FR GB IT LI LU NL SE**

(56) References cited:
**GB-A-1 565 966**
**GB-A-2 067 991**

(73) Proprietor: **GLAXO GROUP LIMITED**
**Clarges House 6-12 Clarges Street**
**London W1Y 8DH (GB)**

(72) Inventor: ***Crookes, Derek Leslie***
**29 Hazeldell**
**Watton-At-Stone Hertfordshire (GB)**

(74) Representative: **Marchant, James Ian et al**
**Elkington and Fife High Holborn House 52/54**
**High Holborn**
**London WC1V 6SH (GB)**

**EP 0 055 626 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to a process for the preparation of a furan derivative.

The furan derivative of formula (I)

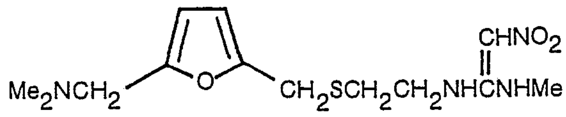

(I)

which is known as ranitidine is disclosed in British Patent Specification No. 1565966 as a potent and selective $H_2$- antagonist.

The present invention provides a process for the preparation of ranitidine of formula (I) which comprises reacting a compound of formula (II)

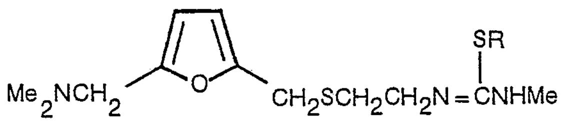

(II)

in which R is a straight or branched $C_{1-6}$ alkyl group optionally substituted by phenyl, with nitromethane at an elevated temperature, for example 80—110°C. Preferably R is methyl, butyl or benzyl, more preferably methyl.

Thus for example, an isothiourea of formula (II) in which R is for example methyl may be heated with nitromethane at about 100°C. Optionally the reaction may be carried out in the presence of a suitable solvent such as acetonitile, N-methylpyrrolidone, a ketone (e.g. methyl isobutyl ketone), an alkanol (e.g. isopropanol or t-butanol) or a hydrocarbon (e.g. toluene). A base, such as an inorganic base (e.g. potassium carbonate) or an amine (e.g. triethylamine), may optionally be present. The reaction is preferably carried out in an inert atmosphere, for example under nitrogen.

The present invention has the advantage over previously described methods for the preparation of ranitidine that the evolution of thiol is significantly reduced or even eliminated. Since it is necessary to prevent the release of such thiols into the environment and this requires the use of specialised equipment which is expensive to run, a process which reduces the amount of thiol evolved offers a significant advantage.

The isothiourea of formula (II) may be prepared from a salt of the thiourea (III)

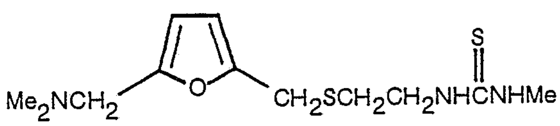

(III)

for example, a hydrochloride salt, by alkylation with an appropriate alkylating agent, such as an alkyl or aralkyl halide (e.g. an alkyl iodide such as methyl iodide). The reaction is carried out in a solvent such as an alkanol (e.g. methanol), and preferably with heating, for example at the reflux temperature of the solvent.

If desired the isothiourea of formula (II) may be isolated in the form of an acid addition salt, for example as an oxalate.

The isothiourea of formula (II) and the thiourea (III) can exhibit tautomerism, and the formulae are intended to cover all tautomers.

If desired the furan derivative of formula (I) once obtained may be converted into an acid addition salt, e.g. a hydrochloride, using conventional methods. Thus, for example appropriate quantities of the free base of formula (I) and an acid, e.g. hydrochloric acid, may be mixed in a suitable solvent(s), e.g. an alcohol such as ethanol, or an ester such as ethyl acetate.

The invention is illustrated by the following examples:

Example 1

N-[2-[[5-[(Dimethylamino)methyl]-2-furanylmethyl]thio]ethyl]-N'-methyl-2-nitro-1,1-ethenediamine

Methyl N'-[2-[[5-[(Dimethylamino)methyl]-2-furanylmethyl]thio]ethyl]-N-methylcarbamimidothioate

A solution of N-[2-[[5-[(dimethylamino)methyl]-2-furanylmethyl]thio]ethyl]-N'-methylthiourea (5.75 g) in methanol (30 ml) was just acidified with hydrogen chloride in ether. The ether was evaporated *in vacuo*, methyl iodide (3.12 g) added and the solution heated under reflux for 1.25 hours. The solution was evaporated *in vacuo*, the oily residue dissolved in water (30 ml) and an excess of anhydrous sodium

carbonate added. The oily suspension was extracted with ethyl acetate (2 × 40 ml), dried ($MgSO_4$), decolourised with charcoal, filtered and evaporated to give the *title compound* (5 g) as an oil.

Found: C, 52.0; H, 7.9; N, 13.9

$C_{13}H_{23}N_3OS_2$ requires: C, 51.8; H, 7.7; N, 14.0%.

The title compound (0.6 g) and oxalic acid (0.5 g) in ethanol (20 ml) yielded the *oxalate salt* (1:2) (0.72 g), m.p. 140—141°.

N-[2-[[5-[(Dimethylamino)methyl]-2-furanylmethyl]thio]ethyl]-N'-methyl-2-nitro-1,1-ethenediamine

To a solution of methyl N'-[2-[[5-[(dimethylamino)methyl]-2-furanylmethyl]thio]ethyl]-N-methylcarbamimidothioate, oxalate (1:2) (4.81 g) in water (10 ml.) was added a solution of anhydrous sodium carbonate (5 g) in water (10 ml) and the oily suspension extracted with ether (2 × 30 ml). The combined ether extracts were dried ($Na_2CO_3$), the mixture filtered and the filtrate evaporated *in vacuo* to give an oil (2.55 g). This was dissolved in nitromethane (6.1 g) and the solution heated at 98—100°C for 22 hr. The mixture was evaporated *in vacuo* and the oily residue re-evaporated with ethanol (2 × 20 ml). The oily residue was dissolved in ethanol (20 ml), the solution treated with decolourising charcoal, the suspension filtered and the filtrate evaporated *in vacuo* to give an oily residue (2.52 g). This was dissolved in 4-methylpentan-2-one (20 ml), and decolourising charcoal added. The suspension was heated to 98—100° and filtered. The filtrate was evaporated *in vacuo* and the oily residue (1.26 g) chromatographed (silica; methanol: 0.88 ammonia 79:1). The appropriate eluate was evaporated *in vacuo* to give an oily residue which was dissolved in 4-methylpentan-2-one (3 ml). The solid which separated was filtered off, washed with 4-methylpentan-2-one and dried to give the *title compound* (0.52 g), m.p. 69—71°, which was not depressed on admixture with a sample prepared according to the method of Example 15 in British Patent Specification No: 1,565,966. T.l.c. silica, ethyl acetate:isopropanol:water:0.88 ammonia (25:15:8:2), Rf 0.3. A second crop of *title compound* (0.12 g) m.p. 65—68° was obtained from the mother liquors.

Example 2

N-[2-[[5-[(Dimethylamino)methyl]-2-furanylmethylthio]ethyl]-N'-methyl-2-nitro-1,1-ethenediamine

A solution of methyl N'-[2-[[5-[(dimethylamino)methyl]-2-furanylmethyl]thio]ethyl]-N-methylcarbamimidothioate oxalate (1:2) (4.81 g) in water (10 ml) was basified with aqueous sodium carbonate. The aqueous solution was extracted with ether. The organic extracts were dried ($MgSO_4$), and evaporated to give an oil (2.45 g) which was heated at reflux with nitromethane (2.16 ml) in toluene (10 ml) for 85 h. The solvent was removed *in vacuo* to leave a brown oil which was chromatographed on silica using methanol:0.880 ammonia (80:1) to give an oil which was crystallised from 4-methylpentan-2-one to give the *title compound* as a fawn solid (0.12 g), m.p. 67-69° which was not depressed on admixture with a sample prepared according to the method of Example 15 in British Patent Specification No. 1,565,966.

Example 3

N-[2-[[5-[(Dimethylamino)methyl]-2-furanylmethyl]thio]ethyl]-N'-methyl-2-nitro-1,1-ethenediamine

A solution of N-[2-[[5-(dimethylaminomethyl)-2-furanylmethyl]thio]ethyl]-N'-methyl thiourea (7 g) in methanol (60 ml) was slightly acidified with ethereal hydrogen chloride. The diethyl ether was evaporated under reduced pressure and further methanol (10 ml), and butyl iodide (5.6 g) added. The reaction mixture was then heated under reflux for 4.5 h and evaporated *in vacuo* to leave a brown oil which was dissolved in water (40 ml). Excess sodium carbonate was added and the suspension extracted with ethyl acetate. The extract was dried ($MgSO_4$) and evaporated to leave a brown oil (4.9 g) which was dissolved in nitromethane (8.5 g). This solution was heated at 100°C for 22 h and evaporated *in vacuo* to give a brown oil which was re-evaporated with ethanol (2 × 20 ml) to leave a brown oil. This oil was chromatographed (Silica:dichloromethane:ethanol:ammonia — 50:8:1) to give a brown gum. This gum was further purified by chromatography (silica methanol:ammonia — 80:1) to give a light yellow oil which was dissolved in 4-methylpentan-2-one (6 ml). The solid which separated was filtered off, washed with 4-methylpentan-2-one and dried to give the *title compound* (0.52 g), m.p. 69—71° which was not depressed on admixture with a sample prepared according to the method of Example 15 in British Patent Specification No. 1,565,966.

Example 4

N-[2-[[5-[(Dimethylamino)methyl]-2-furanylmethyl]thio]ethyl]-N'-methyl-2-nitro-1,1-ethenediamine

a) A suspension of methyl N'-[2-[[5-[(dimethylamino)methyl]-2-furanylmethyl]thio]ethyl]-N-methylcarbamimidothioate (compound A) (0.6 g) and anhydrous potassium carbonate (0.3 g) in nitromethane (6 ml) was heated at reflux for 7 h. T.l.c. of the reaction mixture on silica using ethyl acetate:isopropanol:water:0.88 ammonia (25:15:8:2) showed the presence of the *title compound* Rf 0.3 in good yield.

b) The above preparation was repeated using triethylamine (0.5 g), compound A (1.5 g) and nitromethane (15 ml) at reflux for 7 h. T.l.c. showed the presence of the *title compound* Rf 0.3 in high yield.

**Claims**

1. A process for the preparation of ranitidine of formula (I)

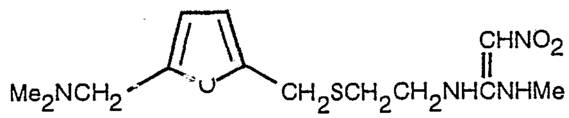

(I)

which comprises reacting a compound of formula (II)

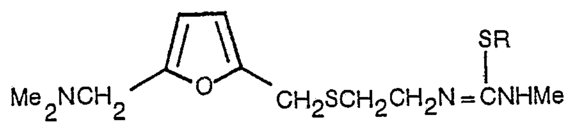

(II)

in which R is a straight or branched $C_{1-6}$ alkyl group optionally substituted by phenyl, with nitromethane at an elevated temperature.

2. A process as claimed in claim 1 wherein the temperature is from 80 to 110°C.
3. A process as claimed in claims 1 or 2 wherein R is methyl, butyl or benzyl.
4. A process as claimed in claim 3 wherein R is methyl.
5. A process as claimed in claim 3 or 4 wherein the temperature is about 100°C.
6. A process as claimed in any of claims 1 to 5 carried out in the presence of a suitable solvent.
7. A process as claimed in any of claims 1 to 6 carried out in an inert atmosphere.
8. A process as claimed in any of claims 1 to 7 carried out in the presence of a base.
9. A process as claimed in any of claims 1 to 8 in which the compound of formula (1) is converted into an acid addition salt.
10. A process as claimed in claim 9 in which the acid addition salt is the hydrochloride.

**Patentansprüche**

1. Verfahren zur Herstellung von Ranitidin der Formel (I)

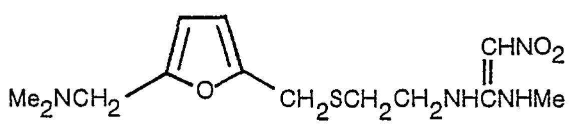

(I)

dadurch gekennzeichnet, daß man eine Verbindung der Formel (II)

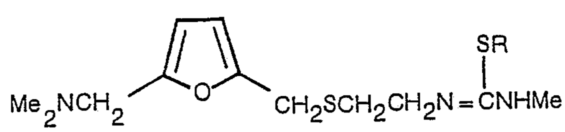

(II)

worin R eine geradkettige oder verzweigt $C_{1-6}$-Alkylgruppe, die gegebenenfalls durch Phenyl substituiert sein kann, bedeutet, mit Nitromethan bei erhöhter Temperatur umsetzt.

2. Verfahren nach Anspruch 1, dadurch gekennzeichnet, daß die Temperatur 80 bis 110°C beträgt.
3. Verfahren nach Anspruch 1 oder 2, dadurch gekennzeichnet, daß R Methyl, Butyl oder Benzyl bedeutet.
4. Verfahren nach Anspruch 3, dadurch gekennzeichnet, daß R Methyl bedeutet.
5. Verfahren nach Anspruch 3 oder 4, dadurch gekennzeichnet, daß die Temperatur ungefähr 100°C beträgt.
6. Verfahren nach einem der Ansprüche 1 bis 5, dadurch gekennzeichnet, daß es in Gegenwart eines geeigneten Lösungsmittels durchgeführt wird.
7. Verfahren nach einem der Ansprüche 1 bis 6, dadurch gekennzeichnet, daß es in einer Inertatmosphäre durchgeführt wird.
8. Verfahren nach einem der Ansprüche 1 bis 7, dadurch gekennzeichnet, daß es in Anwesenheit einer Base durchgeführt wird.
9. Verfahren nach einem der Ansprüche 1 bis 8, dadurch gekennzeichnet, daß die Verbindung der Formel (I) in ein Säureadditionssalz überführt wird.

10. Verfahren nach Anspruch 9, dadurch gekennzeichnet, daß das Säureadditionssalz das Hydrochlorid ist.

**Revendications**

1. Procédé de préparation de ranitidine de formule (I)

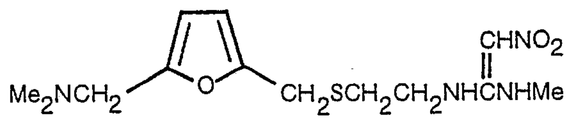

(I)

qui consiste à faire réagir un composé de formule (II)

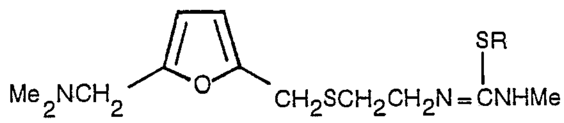

(II)

dans laquelle R est un radical alkyle linéaire ou ramifié en $C_{1-6}$ facultativement substitué par un phényle, avec le nitrométhane à une température élevée.

2. Procédé selon la revendication 1, dans lequel la température est de 80 à 110.

3. Procédé selon la revendication 1 ou 2, dans lequel R est le radical méthyle, butyle ou benzyle.

4. Procédé selon la revendication 3, dans lequel R est le radical méthyle.

5. Procédé selon la revendication 3 ou 4, dans lequel la température est d'environ 100°C.

6. Procédé selon l'une quelconque des revendications 1 à 5, qu'on met en oeuvre en présence d'un solvant convenable.

7. Procédé selon l'une quelconque des revendications 1 à 6 qu'on met en oeuvre en atmosphère inerte.

8. Procédé selon l'une quelconque des revendications 1 à 7 qu'on met en oeuvre en présence d'une base.

9. Procédé selon l'une quelconque des revendications 1 à 8, dans lequel on convertit le composé de formule (I) en un sel d'addition avec un acide.

10. Procédé selon la revendication 9, dans lequel le sel d'addition avec un acide est le chlorhydrate.